(19) Europäisches Patentamt
European Patent Office
Office européen des brevets

(11) Numéro de publication: 0 135 432 B1

# (12) FASCICULE DE BREVET EUROPEEN

(45) Date de publication du fascicule du brevet: **01.06.88**

(21) Numéro de dépôt: **84401675.8**

(22) Date de dépôt: **16.08.84**

(51) Int. Cl.⁴: **A 61 K 31/235**, C 07 C 69/76, C 07 C 65/21, C 07 C 65/01, C 07 C 69/92 // C07C33/20, C07C121/52, C07C79/22, C07C79/35, C07C87/50

## (54) Dérivés de l'acide benzoïque, procédé de préparation et application à titre de médicaments désinfectants ou de conservateurs.

(30) Priorité: **18.08.83 FR 8313445**

(43) Date de publication de la demande: **27.03.85 Bulletin 85/13**

(45) Mention de la délivrance du brevet: **01.06.88 Bulletin 88/22**

(84) Etats contractants désignés: **AT BE CH DE FR GB IT LI LU NL SE**

(56) Documents cités:
**FR - A - 2 241 296**
**FR - A - 2 272 647**
**GB - A - 1 050 136**

(73) Titulaire: **SANOFI, 40, Avenue George V, F-75008 Paris (FR)**

(72) Inventeur: **Demarne, Henri, Le Florence 65 avenue Major Flandres, F-34000 Montpellier (FR)**
Inventeur: **Filhol, Robert, Château d'Alco Bâtiment 7 rue des Avants-Monts, F-34000 Montpellier (FR)**
Inventeur: **Mossé, Madeleine, La Chanterelle Chemin du Réservoir du Montmaur, F-34100 Montpellier (FR)**

(74) Mandataire: **Gillard, Marie-Louise et al, Cabinet Beau de Loménie 55, Rue d'Amsterdam, F-75008 Paris (FR)**

**EP 0 135 432 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne des dérivés de l'acide benzoïque de formule générale

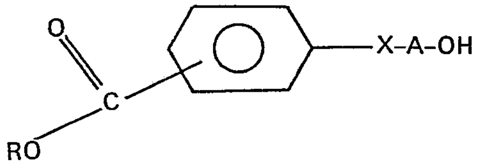

(I)

dans laquelle:

- A représente une chaîne alkyle droite ou ramifiée comprenant de 5 à 10 atomes de carbone;
- X représente l'atome d'oxygène ou une liaison directe;
- R représente l'hydrogène ou un groupement alkyle de 2 à 6 atomes de carbone éventuellement substitué par une fonction alcool.

L'invention concerne aussi un procédé pour l'obtention de ces dérivés et les compositions à usage antiseptique les contenant.

La présente invention concerne également l'utilisation, dans des compositions à usage antiseptique, des sels convenables des composés de formule (I).

Ces composés présentent une activité antimicrobienne, ils peuvent notamment être utilisés comme médicaments antiseptiques à usage humain ou vétérinaire ou comme désinfectants sur des surfaces inertes. Ils peuvent également être utilisés comme conservateurs.

Un procédé de préparation d'acides benzoïques, substitués en para par un groupement hydroxyalcoxy est décrit dans le brevet GB 1,050,136. Aucune indication quant à l'utilisation de tels composés n'est fournie.

Les composés (I), dans lesquels X représente une liaison directe, peuvent être préparés selon la méthode décrite dans J. Med. Chem., 1983, vol. 26, pages 335-341 à partir d'un acide toluique.

On peut également les préparer à partir des nitrophénylalcanols ou des nitrophénoxyalcanols (IV). Par hydrogénation catalytique de (IV), on obtient les dérivés de l'aniline correspondants (V), puis par addition du nitrite de sodium en milieu acide, les diazoniums. L'action du cyanure cuivreux conduit ensuite, par la réaction de Sandmeyer, aux dérivés du benzonitrile (VI). Enfin, les composés (I) sont synthétisés par des réactions connues en elles-mêmes et éventuellement transformés en un sel convenable.

(II) 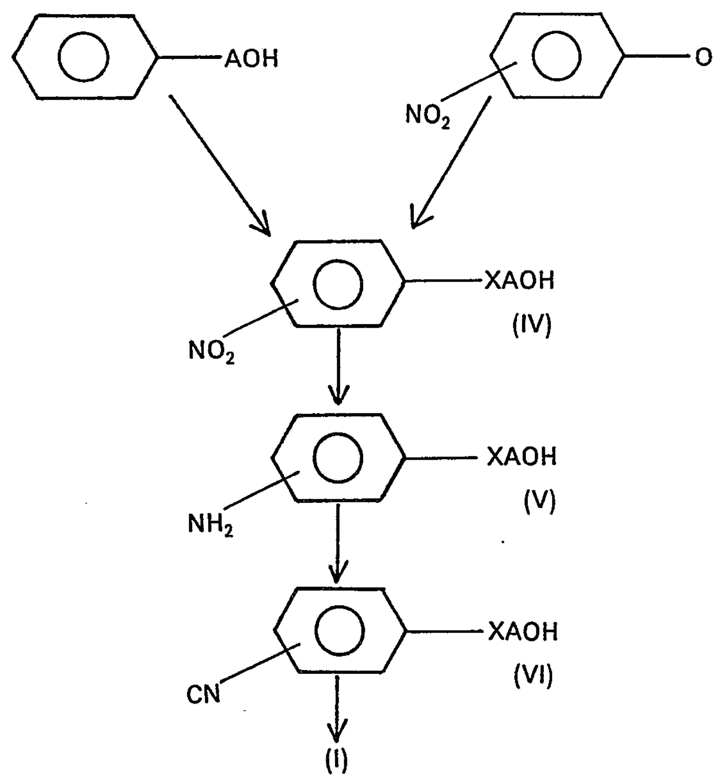 (III)

(IV)

(V)

(VI)

(I)

Lorsque X représente une liaison directe, on prépare le nitrophénylalcanol (IV) à partir du phénylalcanol (II). Au préalable, on protège le groupement hydroxyle par acétylation au moyen du chlorure d'acétyle. Après la nitration par l'acide nitrique fumant, l'alcool est libéré par action du méthanol chlorhydrique.

Les phénylalcanols (II) sont accessibles commercialement lorsqu'il s'agit d'alcools linéaires. Sinon, ils peuvent être préparés par diverses méthodes. Par exemple, les phénylalcanols secondaires sont préparés à partir d'un phénylacétaldéhyde par action d'un dérivé magnésien suivi d'une hydrolyse:

$$C_6H_5-CH_2-CHO \xrightarrow[2°/H_2O]{1°/R_1MgBr} C_6H_5-CH_2-\underset{\displaystyle OH}{\underset{|}{C}H}-R_1$$

(II)'

$R_1$: alkyle de 1 à 8 carbones

Les phénylalcanols ramifiés primaires sont obtenus à partir du cyanure de benzyle:

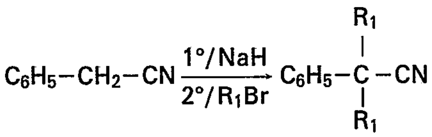


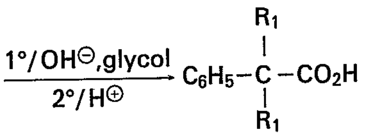


(VII)

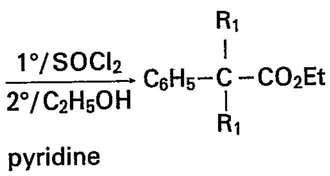


(VIII)

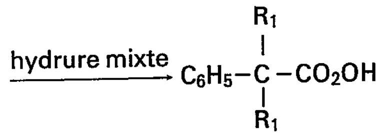


(II)″

Après avoir fait réagir l'hydrure de sodium en milieu anhydre, on peut additionner un halogénure d'alkyle, par exemple le bromure, pour obtenir un phényl-acétonitrile dialkylé symétrique. La transformation de ce composé en acide s'effectue par action d'une base en milieu alcoolique, suivie d'une acidification.

L'action du chlorure de thionyle puis de l'alcool éthylique en milieu anhydre en présence d'un catalyseur, par exemple la pyridine ou la diméthylaminopyridine, permet d'obtenir l'ester éthylique (VII). On prépare ensuite l'alcool correspondant (II)″ par réduction à l'aide d'un hydrure mixte dans un solvant anhydre.

Lorsque X représente l'oxygène, on prépare le nitrophénoxyalcanol (IV) à partir du nitrophénol (III). L'action sur (III) d'un dihalogénure d'alkyle en milieu basique permet d'obtenir un halogénure de nitrophénoxyalkyle (IX). Ce produit est acétylé en milieu acide puis (IV) est libéré par saponification.

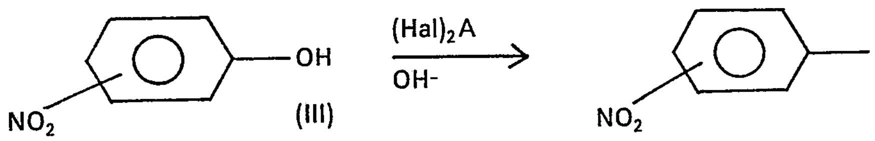


(III) (IX)

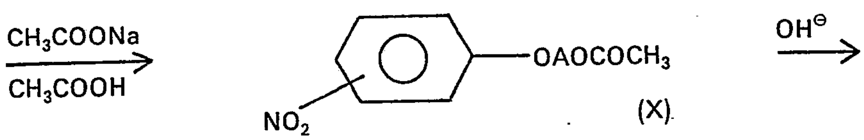


(X) (IV)

Dans tout les cas, les composés (I) où R = alkyle ou hydroxyalkyle sont obtenus à partir des composés (I) où R = H par les procédés habituels d'estérification et en particulier par action de l'alcool R-OH sur l'acide en présence d'acide fort comme catalyseur ou par action d'un halogénure R Hal sur le sel de sodium de l'acide.

Les exemples suivants illustrent l'invention sans toutefois la limiter. Lorsque le produit obtenu est sous forme d'huile, il est caractérisé par son spectre de résonance magnétique nucléaire (RMN). Celui-ci est enregistré à 60 MHz dans le deutérochloroforme, en prenant comme étalon interne l'hexaméthyldisiloxane.

Pour décrire le spectre, les abréviations suivantes sont utilisées:

S : singulet, D : doublet, T : triplet, Q : quadruplet, M : multiplet, J : constante de couplage.

Les exemples 1 à 5 ci-après illustrent la mise en œuvre des procédés décrits ci-dessus. Seuls les composés du tableau I sont des composés selon l'invention.

Exemple 1

Préparation de l'acide (hydroxy-3 propyl)-4 benzoïque: SR 41323.

a) (Nitro-4 phényl)-3 propanol-1.

A 171,5 g de phényl-3 propanol-1, on ajoute en une 1 h sous agitation, 95 ml de chlorure d'acétyle. On chauffe 2 h à reflux, l'acide chlorhydrique qui se dégage et l'excès de chlorure d'acétyle sont éliminés. Lorsque le milieu réactionnel est revenu à la température ambiante, on le verse goutte à goutte, en agitant, sur 800 ml d'acide nitrique fumant (d = 1,49) refroidi à -25°C, l'addition dure 1 h pendant laquelle la température est maintenue entre -15°C et -20°C. On verse ensuite sur 1,5 l d'eau additionnée de glace pilée puis on extrait 3 fois à l'éther, lave 3 fois à l'eau, 3 fois avec une solution de carbonate de sodium à 10%, puis 3 fois à l'eau. Les phases éthérées sont séchées sur sulfate de magnésium puis évaporées à sec sous pression réduite. Le résidu est repris dans 800 ml de méthanol puis on fait barboter de l'acide chlorhydrique gazeux 1 h à 0°C, on chauffe ensuite pendant 14 h à reflux. Après évaporation

du solvant, on reprend dans le mélange eau-éther, décante la phase aqueuse, lave 2 fois à l'eau, 3 fois avec une solution saturée de bicarbonate de sodium puis 3 fois à l'eau; la phase éthérée est ensuite séchée sur sulfate de magnésium, puis évaporée à sec sous pression réduite.

On obtient 259 g d'une huile orangée que l'on purifie par chromatographie sur 3 kg de gel de silice dans le chloroforme; on récupère 218 g d'huile orangée; Rendement 95%.

b) (Hydroxy-3 propyl)-4 aniline

218 g de (nitro-4 phényl)-3 propanol-1 sont dissous dans 500 ml de méthanol, on ajoute 10 g de palladium à 10% sur charbon préalablement humectés avec 10 ml d'eau. L'hydrogénation s'effectue sous une pression de 40 bars et sous agitation, elle dure 1 h 30. On filtre ensuite sur célite, rince au méthanol, évapore à sec sous pression réduite pour obtenir 168 g d'une huile marron. Celle-ci est purifiée par 3 chromatographies successives sur 6 kg d'alumine au total, en utilisant le dichlorométhane comme éluant. On obtient 49,2 g d'une poudre marron clair; Fc = 43-45°C; Rendement: 27%.

c) (Hydroxy-3 propyl)-4 benzonitrile

49,07 g du produit obtenu précédemment sont versés dans 87 ml d'acide chlorhydrique concentré additionné de 400 g de glace pilée. En maintenant la température entre 0°C et 5°C, on ajoute goutte à goutte une solution de 23,15 g de nitrite de sodium dans 80 ml d'eau puis, après 10 min d'agitation, on neutralise avec 300 ml de solution à 10% de carbonate de sodium.

On prépare par ailleurs une solution de cyanure cuivreux: 40,35 g de chlorure cuivreux sont mis en suspension dans 150 ml d'eau, on ajoute une solution de 54 g de cyanure de sodium dans 80 ml d'eau. On observe un dégagement de chaleur, le chlorure cuivreux se dissout, la solution est décolorée. A cette solution refroidie à 0°C et additionnée de 200 ml de benzène, on ajoute goutte à goutte pendant 40 min en agitant violemment, la solution de diazonium refroidie à 0°C. Après 40 min d'agitation supplémentaire, on laisse revenir à température ambiante, sous agitation, puis on chauffe à 50°C sans agiter et on ramène à température ambiante.

On extrait 3 fois à l'éther, lave 2 fois à l'eau, puis avec une solution saturée de chlorure de sodium. Les phases éthérées sont séchées sur sulfate de magnésium et évaporées à sec sous pression réduite. On obtient 51 g d'une huile marron foncé. Celle-ci est purifiée par chromatographie sur 1500 g de gel de silice, la colonne est préparée dans du toluène et l'éluant est un mélange toluène-éther (9/1 en volume). On obtient 41,6 g de produit pur sous forme d'huile rouge; Rendement : 79%.

Le produit est caractérisé par son spectre de RMN:

2H entre 1,7 et 2,2 ppm (M, $-CH_2-CH_2-CH_2-OH$)
1H à 2,4 ppm (S, -OH)
2H à 2,8 ppm (T, J = 7 Hz, $CN-C_6H_4-CH_2-CH_2-$)
2H à 3,6 ppm (T, J = 6 Hz, $-CH_2-CH_2-OH$)
2H à 7,3 ppm (D, J = 9 Hz, H ortho $CH_2$)
2H à 7,6 ppm (D, J = 9 Hz, H ortho CN)

d) SR 41323

41 g du produit précédant sont dissous dans 150 ml d'alcool à 95°, on additionne 15 g de soude puis on chauffe à reflux 17 h sous agitation, après refroidissement, on évapore à sec sous pression réduite, le résidu est repris dans l'eau et acidifié à pH voisin de 1 par de l'acide chlorhydrique concentré. On extrait 2 fois à l'éther, lave 2 fois à l'eau puis avec une solution saturée de chlorure de sodium. Les phases éthérées sont ensuite séchées sur sulfate de magnésium et évaporées à sec sous pression réduite. Les cristaux jaunes obtenus sont recristallisés dans un mélange étherhexane.

On obtient 33,7 g de poudre crème; Fc = 138-141°C.

Exemple 2

Préparation d'(Hydroxy-3 propyl)-4 benzoate d'éthyle: SR 41324.

13,5 g du produit précédemment obtenu (SR 41324) sont dissous dans 200 ml d'éthanol absolu, on ajoute goutte à goutte, en agitant, 6 ml de chlorure de thionyle. Lorsque le milieu réactionnel est revenu à la température ambiante, on chauffe à reflux 6 h sous agitation puis on abandonne une nuit à température ambiante. Les solvants sont évaporés sous pression réduite, puis on extrait 3 fois à l'éther, lave 2 fois avec une solution saturée de bicarbonate de sodium, 2 fois à l'eau puis avec une solution saturée de chlorure de sodium. Les phases éthérées sont ensuite séchées sur sulfate de magnésium et évaporées à sec sous pression réduite.

On obtient 15,43 g correspondant au produit attendu caractérisé par son spectre de RMN.

3H à 1,3 ppm (T, J = 7 Hz, $-CO_2-CH_2-CH_3$)
3H entre 1,5 et 2,2 ppm (massif, $HO-CH_2-CH_2-CH_2$)
2H à 2,7 ppm (T, J = 7 Hz, $HO-CH_2-CH_2-CH_2-C_6H_4$)
2H à 3,6 ppm (T, J = 6 Hz, $HO-CH_2-CH_2-CH_2-$)
2H à 4,3 ppm (Q, J = 7 Hz, $-CO_2-CH_2-CH_3$)
2H à 7,2 ppm (D, J = 9 Hz, H ortho $CH_2$)
2H à 7,9 ppm (D, J = 9 Hz, H ortho $CO_2$)

Exemple 3

Préparation de l'acide (hydroxy-2 butyl)-4 benzoïque: CM 41074.

a) Phényl-1 butanol-2

A 2,92 g de magnésium en tournures, on ajoute sous azote, goutte à goutte, à une vitesse suffisante pour maintenir un léger reflux, une solution de 7,5 ml de bromure d'éthyle dans 50 ml d'éther anhydre. Toujours sous azote, on agite pendant 2 h à température ambiante, puis on ajoute goutte à goutte 9,4 ml de phénylacétaldéhyde et on laisse 2 h à température ambiante sous agitation.

On décompose ensuite sur 200 ml de chlorure d'ammonium à 20% refroidi à 0°C et on extrait 3

fois à l'éther. Après 3 lavages à l'eau, les phases éthérées sont séchées sur sulfate de magnésium et évaporées à sec sous pression réduite. On obtient 12,2 g d'une huile légèrement jaune.

b) CM 41074

En utilisant ensuite les procédés décrits à l'exemple 1, on prépare le CM 41074 qui est recristallisé dans un mélange étherhexane; Fc = 103–106°C.

Exemple 4

Préparation d'(Hydroxy-2 butyl)-4 benzoate d'éthyle: CM 41075.

Ce produit est préparé à partir de l'acide CM 41074, il est caractérisé par son spectre de RMN.

3H à 0,9 ppm (T, J = 7 Hz $CH_3$-$CH_2$-CH (OH)-$CH_2$-)
6H entre 1,1 et 1,7 ppm (massif, $CH_3$-$CH_2$-CH(OH), -$CH_2$-$CH_2$-$CH_3$)
2H entre 2,6 et 2,9 ppm (M, CH(OH)-$CH_2$-$C_6H_4$-)
1H entre 3,5 et 3,9 ppm (M, -$CH_2$-CH(OH)-$CH_2$)
2H à 4,3 ppm (Q, J = 7 Hz, -$CO_2$-$CH_2$-$CH_3$)
2H à 7,3 ppm (D, J = 9 Hz, H ortho $CH_2$)
2H à 8 ppm (D, J = 9 Hz, H ortho $CO_2$)

Exemple 5

Préparation de l'acide (hydroxy-4 butyloxy)-4 benzoïque: CM 40841.

a) (Nitro-4 phénoxy)-1, bromo-4 butane

A une solution de nitro-4 phénol dans 275 ml d'eau, on ajoute 83 ml de dibromo-1,4 butane puis, goutte à goutte, 49,5 ml de soude 10 N sous agitation. On chauffe à reflux sous agitation pendant 24 h.

Après refroidissement, on extrait 3 fois à l'éther, lave 6 fois à la soude normale, puis 3 fois à l'eau. Les phases éthérées sont séchées sur sulfate de sodium et évaporées, on filtre l'insoluble. Le filtrat est évaporé à sec et le résidu est tiré sous vide (0,05 mm Hg). Après trituration dans l'hexane, on obtient des cristaux que l'on filtre, lave à l'hexane et sèche sous vide au dessiccateur.

On obtient 75 g d'un produit pâteux de couleur crème; Rendement 55%.

b) (Nitro-4 phénoxy)-1 acétyloxy-4 butane

75 g du produit précédent sont dissous dans 80 ml d'acide acétique glacial, on ajoute 45 g d'acétate de sodium anhydre, puis on chauffe à reflux pendant 15 h sous agitation. Le mélange réactionnel est versé sur 1 l d'eau glacée additionnée de 500 ml d'éther et on neutralise à pH 7,5 par du carbonate de sodium solide. Après 3 extractions à l'éther et 3 lavages à l'eau, les phases éthérées sont séchées sur sulfate de magnésium et évaporées à sec, le résidu est tiré sous vide.

On obtient m = 70 g d'une huile orangée; Rendement: 100%.

c) (Nitro-4 phénoxy)-1 butanol-4

70 g du produit obtenu précédemment sont mis en solution dans 300 ml de méthanol, on ajoute 30 ml de soude 10 N puis on chauffe à reflux pendant 4 h sous agitation. Après avoir évaporé le méthanol, le résidu est repris dans un mélange eau-éther, on extrait 3 fois à l'éther, lave 3 fois avec une solution saturée de chlorure de sodium.

Les phases éthérées sont ensuite séchées sur sulfate de magnésium et évaporées à sec. Les cristaux formés sont triturés dans l'hexane, filtrés, lavés à l'hexane et séchés sous vide au dessiccateur.

On obtient 48,8 g de cristaux, légèrement jaunes; Fc = 53–55°C.

d) CM 40841

En opérant comme dans l'exemple 1, on prépare ensuite les composés suivants:

- (Hydroxy-4 butyloxy)-4 aniline.
Fc = 56–58°C.
- (Hydroxy-4 butyloxy)-4 benzonitrile.
Fc = 54–58°C.
- CM 40841.
Fc = 143–145°C après recristallisation dans l'éther.

En utilisant des procédés de préparation analogues, on prépare les composés, selon l'invention, décrits dans le tableau I ci-dessous. Ils sont caractérisés par leur spectre de RMN ou leur point de fusion mesuré après recristallisation dans un mélange éthanol-hexane.

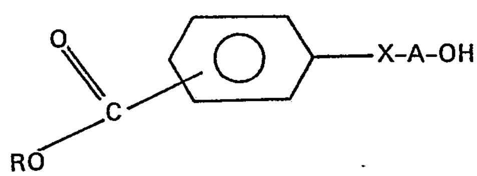

(I)

Tableau I

| N° du produit | Position | X | A – OH | R | Préparé selon exemple | Point de fusion |
|---|---|---|---|---|---|---|
| SR 41945 | p | – | $(CH_2)_5OH$ | H | 1 | 104–106°C |
| CM 41093 | p | – | $CH_2CH(OH)$-$nC_3H_7$ | H | 3 | 128–130°C |
| CM 40937 | p | O | $(CH_2)_5OH$ | H | 5 | 105–115°C |
| SR 41709 | p | – | $(CH_2)_5OH$ | $C_2H_5$ | 2 | RMN |
| CM 41094 | p | – | $CH_2CH(OH)$-$nC_3H_7$ | $C_2H_5$ | 4 | RMN |
| CM 40939 | p | O | $(CH_2)_5OH$ | $C_2H_5$ | 5 | RMN |

Spectres de RMN des produits selon l'invention:

- SR 41709:

9H entre 1,1 et 1,9 ppm (massif, HO-$CH_2$-$(CH_2)_3$-$CH_2$ et $CO_2$-$CH_2CH_3$)
2H à 2,6 ppm (T, J = 7 Hz, -$CH_2CH_2$-$C_6H_4$)
2H à 3,6 ppm (T, J = 6 Hz, HO-$CH_2$-$CH_2$-)
2H à 4,3 ppm (Q, J = 7 Hz, -$CO_2$-$CH_2$-$CH_3$)
2H à 7,2 ppm (D, J = 9 Hz, H ortho $CH_2$)
2H à 8 ppm (D, J = 9 Hz, H ortho $CO_2$).

- SR 41094:

11H entre 0,7 et 1,7 ppm (massif, $CH_3$-$CH_2$-$CH_2$-CH(OH), -$CO_2$-$CH_2$-$CH_3$)
2H entre 2,6 et 2,9 ppm (M, -CH(OH)-$CH_2$-$C_6H_4$)
1H entre 3,6 et 4 ppm (M, -$CH_2$-CH(OH)-$CH_2$)
2H à 4,3 ppm (Q, J = 7 Hz, -$CO_2$-$CH_2$-$CH_3$)
2H à 7,3 ppm (D, J = 9 Hz, H ortho $CH_2$)
2H à 8 ppm (D, J = 9 Hz, H ortho $CO_2$).

- CM 40939:

3H à 1,3 ppm (T, J = 7 Hz, -$CO_2$-$CH_2$-$CH_3$)
6H entre 1,4 et 1,9 ppm (massif, HO-$CH_2$-$CH_2$-$CH_2$-$CH_2$-$CH_2$-O-)
2H à 3,6 ppm (T, J = 6 Hz, -$CH_2$-$CH_2$-O-$C_6H_4$)
2H à 4 ppm (T, J = 6 Hz, HO-$CH_2$-$CH_2$-)
2H à 4,3 ppm (Q, J = 7 Hz, $CO_2CH_2CH_3$)
2H à 6,9 ppm (D, J = 9 Hz, H méta $CO_2$)
2H à 8 ppm (D, J = 9 Hz, H ortho $CO_2$).

L'activité bactéricide des produits selon l'invention a été étudiée sur différentes souches par la méthode décrite ci-dessous:

Un inoculum bactérien est mis en contact avec différentes dilutions du produit à tester et, ce, pendant un temps limité. A la fin du contact, une partie aliquote du mélange suspension bactérienne/produit est déposée à la surface d'un milieu de culture gélosé contenant un neutralisant de l'activité antibactérienne du produit.

La concentration bactéricide retenue est la concentration minimale de produit à partir de laquelle les bactéries ne poussent plus. Cette concentration est exprimée en µg/ml.

Les souches bactériennes choisies pour l'étude sont:

1 - Escherichia coli CNCM 54125;
2 - Klebsiella pneumoniae capsulée R030;
3 - Pseudomonas aeruginosa CNCM A22;
4 - Streptococcus faecalis CNCM 5855;
5 - Staphylococcus aureus CNCM 53154.

La deuxième est entretenue sur milieu Worgel Ferguson, les autres sur Tryptic Soy Agar-Difco (TSA).

Après 24 h de culture à 37°C, on récolte la pousse microbienne à l'aide de billes de verre et de 10 ml de diluant contenant 1 g de tryptone et 8,5 g de chlorure de sodium dans 1000 ml d'eau distillée. On agite la suspension formée et on mesure au spectrophotomètre le pourcentage de transmission de la lumière à 620 nm:

- Souche 1: 70%;
- Souche 2: 80%;
- Souche 3: 70%;
- Souche 4: 60%;
- Souche 5: 60%.

L'inoculum bactérien correspond à une suspension au 1/20e de cette suspension bactérienne.

Des plaques comportant des cupules reçoivent différentes dilutions du produits à étudier. Ces dilutions du produit à étudier sont mises en contact avec les différentes suspensions bactériennes à l'aide d'un inoculateur à site multiple type Steers. Après 20 min de contact, des parties aliquotes sont transférées à l'aide de cet inoculateur à la surface d'un milieu gélosé (TSA) placé dans des boîtes de Petri contenant un neutralisant de l'activité, à savoir 20 g de lubrol W, 2,5 g de Tween 80® et 2,5 g de thiosulfate de sodium dans 1000 ml de TSA (Difco). Un témoin de l'efficacité du neutralisant est réalisé pour chaque produit étudié en déposant à la surface du milieu de culture une partie aliquote de la dilution du produit à étudier. Après séchage, l'inoculum correspondant est déposé au même endroit. Un témoin inoculum est réalisé sur milieu gélosé avec et sans neutralisant. La lecture se fait après 48 h d'incubation à 37°C.

Les résultats sont rassemblés dans le tableau II ci-dessous.

Tableau II

Concentration minimale bactéricide (CMB) en µg/ml

| N° du produit | Souches bactériennes | | | | |
|---|---|---|---|---|---|
| | 1 | 2 | 3 | 4 | 5 |
| SR 41945 | 500 | 750 | 250 | 500 | 500 |
| CM 40937 | 600 | 200 | 400 | 800 | 600 |
| CM 40939 | 500 | 800 | 1000 | 1000 | 1000 |
| SR 41709 | 250 | 100 | 600 | 400 | 100 |

Les résultats montrent que les produits selon l'invention présentent un large spectre d'activité sur les souches bactériennes testées.

L'activité antifongique des produits selon l'invention a aussi été déterminée selon la méthode des dilutions sériées en milieu gélosé.

Des solutions mères des différents produits sont réalisées à l'aide de tétraglycol à 20%. A partir de ces solutions mères, on réalise une gamme de dilutions selon une progression géométrique de raison 1/2. 2 ml de chacune de ces dilutions sont distribués dans des boîtes de Petri et on y

ajoute 1,8 ml de milieu gélosé «Sabouraud Dextrose Agar» (Difco).

L'inoculum est constitué pour les levures d'une dilution au 1/20e d'une culture de 48 h à 27°C en milieu liquide de Sabouraud. Pour les dermatophytes et les contaminants, l'inoculum est préparé par la récolte, à l'aide de 5 ml de milieu de Sabouraud liquide, d'une culture en milieu gélosé de Sabouraud.

L'inoculum est déposé à la surface des boîtes de Petri à l'aide d'un inoculateur à sites multiples. La lecture se fait après 48 h d'incubation à 27°C pour les levures, après 7 jours d'incubation à 27°C pour les dermatophytes et après 4 jours d'incubation à 27°C pour les contaminants. La concentration minimale inhibitrice ou CMI exprimée en µg/ml représente la plus petite concentration pour laquelle aucune pousse ne s'est produite.

Le tableau III rassemble les résultats obtenus avec différents produits selon l'invention.

Les expériences ont été effectuées avec 22 souches de levures, 18 souches de dermatophytes et 8 souches de contaminants. Les résultats (CMI en µg/ml) représentent les valeurs extrêmes obtenues pour chaque catégorie.

Tableau III

Activité antifongique

| N° du produit | Levures | Dermatophytes | Contaminants |
|---|---|---|---|
| SR 41709 | 150-750 | 19-75 | 750 |
| SR 41945 | 750 | 9-75 | 750 |
| CM 40939 | 200-500 | 25 | 200-500 |
| CM 40937 | 100-500 | 50-100 | 100-500 |

Ces résultats montrent que les produits selon l'invention possèdent une bonne activité vis-à-vis des champignons étudiés.

La tolérance des produits selon l'invention a été étudiée chez le cobaye. Les animaux sont tondus de part et d'autre de la ligne médiane du dos, la tonte est entretenue tous les 2 jours. Des lots de 6 animaux reçoivent sur la zone tondue 0,2 ml d'une solution aqueuse ou alcoolique du produit selon l'invention. Lorsque les produits sont en solution alcoolique, un lot témoin d'animaux reçoit l'alcool sur un côté.

Pour l'étude de la tolérance cutanée préliminaire, le traitement est appliqué 1 fois par jour, 6 jours sur 7, durant 3 semaines. Les observations sur le plan cutané portent sur la présence d'érythème, d'éruption cutanée ou d'hyperkératose dont l'intensité est graduée selon un barème déterminé.

L'essai de sensibilisation cutanée est réalisé sur les mêmes animaux après deux semaines de repos. Le traitement dure une semaine, il est identique au précédent. L'évaluation se fait sur les mêmes critères et d'après la même barème que celui utilisé pour la tolérance locale.

On a également recherché si les produits selon l'invention présentent un effet phototoxique ou photoallergique chez le cobaye. La technique mise en œuvre est celle de Unkovic J., Mazue G. et Girard J., dans Sciences et Techniques de l'Animal de laboratoire vol. 8 (3), 149-160 (1983). C'est une adaptation des techniques décrites par Harber L.C. et al, Arch. Dermatol., 1967, vol. 96, p. 646-656 et Vinson L.J. et al, J. Soc. Cosm. Chem., 1966, vol. 17, p. 123-130.

Aucun des produits étudiés n'a montré de mauvais tolérance, d'effet sensibilisant ni d'effet phototoxique ou photoallergique chez le cobaye.

Une évaluation de la toxicité aiguë par voie orale a été réalisée chez la souris. Cette étude a été effectuée sur des souris mâles de souche CD1 prevenant de l'élevage Charles River. Chaque lot était composé de 5 animaux d'un poids corporel variant de 24 à 30 g entretenus dans une même cage.

Les animaux étaient conservés à jeun pendant 6 h avant le traitement. Pour chaque étude, le produit mis en suspension dans une solution de gomme arabique à 10% a été administré par gavage à l'aide d'une sonde oesophagienne.

La nourriture était de nouveau distribuée aux animaux 4 h après le gavage et les animaux étaient gardés en observation pendant une période de 14 jours après l'administration.

Pendant cette période, on note la mortalité dans chacun des lots mis en expérience et lorsque cela est possible on détermine la dose létale 50 (DL 50) en utilisant la méthode de Litchfield et Wilcoxon.

Les résultats suivants, exprimés en mg de substance essayée par kg de poids corporel, ont été obtenus.

CM 40 937 DL 0 > 3000
SR 41 709 DL 0 > 3000
CM 40 939 DL 0 > 3000

Les composés selon l'invention présentent un niveau d'activité intéressant qui se compare favorablement à celui des principales familles d'antiseptiques utilisés dans la pratique.

En outre les produits selon l'invention présentent une activité homogène vis-à-vis des différentes espèces bactériennes étudiées. De plus, ils sont dotés d'une faible toxicité, ils ont montré une bonne tolérance et sont dépourvus d'effet sensibilisant et d'effet phototoxique ou d'effet photoallergique.

Par suite, les produits selon l'invention pourront recevoir de multiples applications comme antiseptiques, conservateurs ou désinfectants.

En particulier, ils pourront être utilisés comme antiseptiques dans les préparations à visées thérapeutiques, par exemple, dans le traitement de l'impétigo, l'acné, les dermatoses infectées, les plaies ouvertes infectées, les infections fermées telles que furoncles, panaris, gales impétiginisées etc. On peut également envisager une utilisation a visée préventive, par exemple, pour la préparation du champ chirurgical, la préparation des mains du chirurgien ou du personnel soignant.

En application vétérinaire, les produits selon l'invention peuvent être utilisés soit comme antiseptiques (par exemple dans la prévention des

mammites) soit comme désinfectants des étables, du matériel ...) de même que dans le domaine agroalimentaire.

Enfin, leur bonne tolérance et leur faible toxicité les autorisent à être utilisés comme conservateurs, non seulement dans le domaine pharmaceutique et cosmétologique mais également dans le domaine agroalimentaire.

Différentes formulations galéniques des produits selon l'invention peuvent être préparées en fonction de l'application choisie.

Exemple 6

| | | |
|---|---|---|
| Soluté antiseptique alcoolique SR 41945 | | 0,5 g |
| Alkyldiméthylcarboxyméthylamine (solution à 30%) | | 0,5 g |
| Condensat d'oxyde d'éthylène et de propylèneglycol L62 | | 1 g |
| Acide lactique ou hydroxyde de sodium | qsp pH 6,5 | |
| Alcool éthylique à 70° | qsp | 100 g |

Exemple 7

Un produit selon l'invention peut être utilisé comme conservateur dans un shampooing:

| | | |
|---|---|---|
| Palmitate de potassium et d'acides aminés | | 20 g |
| Alkylsulfates de sodium | | 2 g |
| Diéthanolamide de coprah | | 5 g |
| Acétate de linolyle | | 0,200 g |
| CM 40937 | | 0,150 g |
| Hydroxyde de sodium | qsp pH 7 | |
| Eau purifiée | qsp | 100 g |

Exemple 8

Un produit selon l'invention peut être utilisé comme conservateur dans une crème émulsion.

| | | |
|---|---|---|
| Huile de Vaseline ® épaisse | | 6 g |
| Mélange d'alcool cétostéarylique oxyméthyléné | | 9 g |
| Phosphate monosodique anhydre | | 0,300 g |
| Tétracémate disodique | | 0,010 g |
| Vaseline® | | 15 g |
| CM 40937 | | 0,100 g |
| Acide phosphorique | qsp pH 4,5 | |
| Eau purifiée | qsp | 100 g |

Exemple 9

Un produit selon l'invention peut être utilisé comme conservateur dans une crème pour utilisation cosmétologique.

| | | |
|---|---|---|
| Collagène | | 0,500 g |
| Carboxypolyméthylène 934 | | 0,400 g |
| Lanoline hydrogènée | | 4 g |
| Perhydrosqualène | | 20 g |
| Monopalmitate de sorbitol Polyoxyméthyléné | | 2 g |
| SR 41709 | | 0,150 g |
| Acide lactique ou hydroxyde de sodium | qsp pH 6,5 | |
| Eau purifiée | qsp | 100 g |

Exemple 10

Préparation antiseptique liquide détergente moussante:

| | | |
|---|---|---|
| CM 40 939 | | 0,3 g |
| Alkyldiméthylcarboxyméthylamine (solution à 30%) | | 15 g |
| Tétracémate disodique | | 0,1 g |
| Propylèneglycol | | 20 g |
| Hydroxyde de sodium | qsp pH 5,8 | |
| Eau purifiée | qsp | 100 g |

Exemple 11

Préparation antiseptique liquide détergente moussante:

| | | |
|---|---|---|
| SR 41 945 | | 0,2 g |
| Paraffine sulfonate de sodium | | 15 g |
| Hydroxyde de sodium ou acide lactique | qsp pH 5,2 | |
| Eau purifiée | qsp | 100 g |

Exemple 12

Désinfectant pour surface inerte:

| | | |
|---|---|---|
| SR 41709 | | 0,5 g |
| Dodécyldiméthylcarboxydiméthylamine | | 20 g |
| Tétracémate disodique | | 2 g |
| Acide lactique | qsp pH 3,5 | |
| Eau purifiée | qsp | 100 g |

Exemple 13

Conservateur pour jus de fruits ou confiture:

SR 41 945 micronisé 0,05%

Exemple 14

Conservateur pour crèmes:

CM 40939 micronisé 0,05%.

**Revendications pour les Etats contractants BE, CH, DF, FR, GB, IT, LI, LU, NL, SE**

1. Dérivés d'acide benzoïque de formule générale

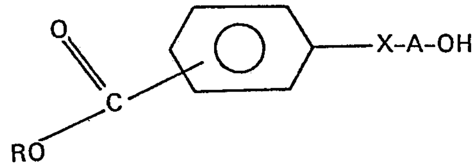

(I)

dans laquelle:

- A représente une chaîne alkyle droite ou ramifiée comprenant de 5 à 10 atomes de carbone;
- X représente l'atome d'oxygène ou une liaison directe;
- R représente l'hydrogène ou un groupement alkyle de 2 à 6 atomes de carbone éventuellement substitué par une fonction alcool, et les sels desdits dérivés.

2. Dérivés d'acide benzoïque selon la revendication 1, caractérisés en ce que X est une liaison directe, R est l'hydrogène ou le groupe éthyle et A-OH est choisi parmi les groupes $(CH_2)_5OH$ et $CH_2CH(OH)$-n $C_3H_7$.

3. Dérivés d'acide benzoïque selon la revendication 1, caractérisés en ce que X est l'oxygène, R est l'hydrogène ou le groupe éthyle et A-OH est le groupe $(CH_2)_5OH$.

4. Procédé pour l'obtention des dérivés d'acide benzoïque selon l'une des revendications 1 à 3, caractérisé en ce qu'il consiste:

1) à soumettre à une hydrogénation catalytique le composé de formule (IV)

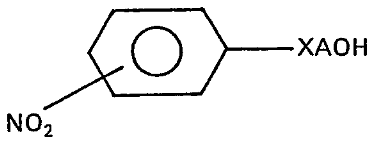
(IV)

dans laquelle X et A sont tels que définis dans la revendication 1, pour former les dérivés de l'aniline correspondants;

2) à ajouter auxdits dérivés de l'aniline du nitrite de sodium en milieu acide pour former les diazoniums correspondants;

3) à transformer lesdits diazoniums en benzonitriles par action du cyanure cuivreux;

4) à transformer les benzonitriles obtenus en composés de formule (I); et

5) éventuellement à transformer ledit composé obtenu en l'un de ses sels.

5. Composition pharmaceutique à usage externe présentant une activité antimicrobienne, caractérisée en ce qu'elle contient une quantité efficace d'un composé selon l'une des revendications 1 à 3.

6. Application des dérivés selon l'une quelconque des revendications 1 à 3 à titre de conservateurs en pharmacie, cosmétologie et agroalimentaire.

7. Produits cosmétiques, caractérisés en ce qu'ils contiennent à titre de conservateurs un dérivé selon l'une quelconque des revendications 1 à 3.

8. Compositions désinfectantes, caractérisées en ce qu'elles contiennent une quantité efficace d'un dérivé selon l'une quelconque des revendications 1 à 3.

**Revendications pour l'Etat contractant AT**

1. Procédé pour l'obtention de dérivés d'acide benzoïque de formule

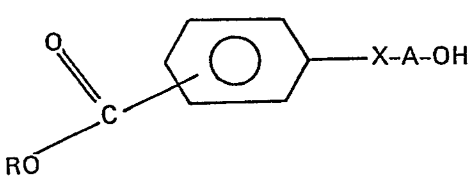
(I)

dans laquelle:
- A représente une chaîne alkyle droite ou ramifiée comprenant de 5 à 10 atomes de carbone;
- X représente l'atome d'oxygène ou une liaison directe;
- R représente l'hydrogène ou un groupement alkyle de 2 à 6 atomes de carbone éventuellement substitué par une fonction alcool, et les sels desdits dérivés, caractérisé en ce qu'il consiste:

1) à soumettre à une hydrogénation catalytique le composé de formule (IV)

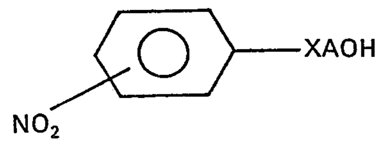
(IV)

dans laquelle X et A sont tels que définis dans la revendication 1, pour former les dérivés de l'aniline correspondants;

2) à ajouter auxdits dérivés de l'aniline du nitrite de sodium en milieu acide pour former les diazoniums correspondants;

3) à transformer lesdits diazoniums en benzonitriles par action du cyanure cuivreux;

4) à transformer les benzonitriles obtenus en composés de formule (I); et

5) éventuellement à transformer ledit composé obtenu en l'un de ses sels.

2. Procédé selon la revendication 1, caractérisé en ce que X est une liaison directe, R est l'hydrogène ou le groupe éthyle et A-OH est choisi parmi les groupes $(CH_2)_5OH$ et $CH_2CH(OH)$-n $C_3H_7$.

3. Procédé selon la revendication 1, caractérisé en ce que X est l'oxygène, R est l'hydrogène ou le groupe éthyle et A-OH est le groupe $(CH_2)_5OH$.

4. Utilisation des dérivés d'acide benzoïque de formule (I) obtenus selon l'une quelconque des revendications 1 à 3, pour la préparation de compositions pharmaceutiques à usage externe, présentant une activité antimicrobienne.

5. Application des dérivés d'acide benzoïque de formule (I), obtenus selon l'une quelconque des revendications 1 à 3, à titre de conservateurs en pharmacie, cosmétologie et agroalimentaire.

6. Utilisation des dérivés d'acide benzoïque de formule (I), obtenus selon l'une quelconque des revendications 1 à 3, pour la préparation de compositions désinfectantes.

**Patentansprüche für die Vertragsstaaten BE, CH, DE, FR, GB, IT, LI, LU, NL, SE**

1. Benzoesäurederivate der allgemeinen Formel

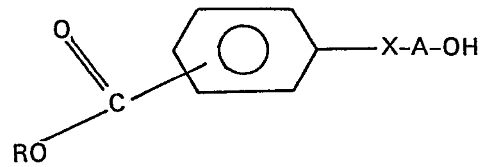
(I)

worin:
- A eine gerade oder verzweigte Alkylkette mit 5 bis 10 Kohlenstoffatomen darstellt;
- X für ein Sauerstoffatom oder eine direkte Bindung steht;
- R Wasserstoff oder eine gegebenenfalls durch eine Alkoholfunktion substituierte Alkylgruppe

mit 2 bis 6 Kohlenstoffatomen darstellt, und die Salze dieser Derivate.

2. Benzoesäurederivate nach Anspruch 1, dadurch gekennzeichnet, dass X eine direkte Bindung ist, R für Wasserstoff oder eine Ethylgruppe steht, und A-OH ausgewählt ist aus den Gruppen $(CH_2)_5OH$ und $CH_2CH(OH)_nC_3H_7$.

3. Benzoesäurederivate nach Anspruch 1, dadurch gekennzeichnet, dass X für Sauerstoff steht, R Wasserstoff oder eine Ethylgruppe ist und A-OH die Gruppe $(CH_2)_5OH$ darstellt.

4. Verfahren zur Darstellung der Benzoesäurederivate nach einem der Ansprüche 1 bis 3, dadurch gekennzeichnet, dass es darin besteht, dass

1) die Verbindung der Formel (IV)

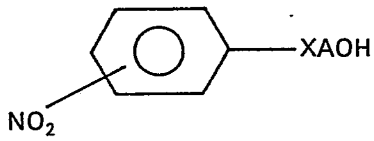

(IV)

worin X und A wie im Anspruch 1 definiert sind, einer katalytischen Hydrierung zur Bildung der entsprechenden Anilinderivate unterzogen wird;

2) zu diesen Anilinderivaten Natriumnitrit in saurem Milieu zur Bildung der entsprechenden Diazoniumverbindungen zugegeben wird;

3) die Diazoniumverbindungen durch Einwirken von Kupferzyanid in Benzonitrile übergeführt werden;

4) die erhaltenen Benzonitrile in Verbindungen der Formel (I) übergeführt werden; und

5) die erhaltene Verbindung gegebenenfalls in eines ihrer Salze übergeführt wird.

5. Pharmazeutische Zusammensetzung zur äusseren Anwendung mit bakteriostatischer Aktivität, dadurch gekennzeichnet, dass sie eine wirksame Menge einer Verbindung nach den Ansprüchen 1 bis 3 enthält.

6. Anwendung der Derivate nach einem der Ansprüche 1 bis 3 als Konservierungsmittel in der Pharmazie, Kosmetologie und in der Agrar-Nahrungsmittelindustrie.

7. Kosmetische Produkte, dadurch gekennzeichnet, dass sie als Konservierungsmittel ein Derivat nach einem der Ansprüche 1 bis 3 enthalten.

8. Desinfektionszusammensetzungen, dadurch gekennzeichnet, dass sie eine wirksame Menge eines Derivats nach einem der Ansprüche 1 bis 3 enthalten.

**Patentansprüche für den Vertragsstaat AT**

1. Verfahren zur Herstellung von Benzoesäurederivaten der allgemeinen Formel

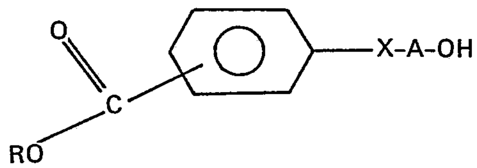

(I)

worin:

- A eine gerade oder verzweigte Alkylkette mit 5 bis 10 Kohlenstoffatomen darstellt;
- X für ein Sauerstoffatom oder eine direkte Bindung steht;
- R Wasserstoff oder eine gegebenenfalls durch eine Alkoholfunktion substituierte Alkylgruppe mit 2 bis 6 Kohlenstoffatomen darstellt, und der Salze dieser Derivate, dadurch gekennzeichnet, dass es darin besteht, dass

1) die Verbindung der Formel (IV)

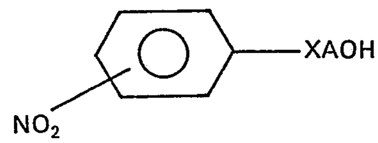

(IV)

worin X und A wie im Anspruch 1 definiert sind, einer katalytischen Hydrierung zur Bildung der entsprechenden Anilinderivate unterzogen wird;

2) zu diesen Anilinderivaten Natriumnitrit in saurem Milieu zur Bildung der entsprechenden Diazoniumverbindungen zugegeben wird;

3) die Diazoniumverbindungen durch Einwirken von Kupferzyanid in Benzonitrile übergeführt werden;

4) die erhaltenen Benzonitrile in Verbindungen der Formel (I) übergeführt werden; und

5) die erhaltene Verbindung gegebenenfalls in eines ihrer Salze übergeführt wird.

2. Verfahren nach Anspruch 1, dadurch gekennzeichnet, dass X eine direkte Bindung ist, R für Wasserstoff oder eine Ethylgruppe steht, und A-OH ausgewählt ist aus den Gruppen $(CH_2)_5OH$ und $CH_2CH(OH)_nC_3H_7$.

3. Verfahren nach Anspruch 1, dadurch gekennzeichnet, dass X für Sauerstoff steht, R Wasserstoff oder eine Ethylgruppe ist und A-OH die Gruppe $(CH_2)_5OH$ darstellt.

4. Verwendung der nach einem der Ansprüche 1 bis 3 erhaltenen Benzoesäurederivate der Formel (I) zur Herstellung von pharmazeutischen Zusammensetzungen für äussere Anwendung welche eine bakteriostatische Aktivität aufweisen.

5. Verwendung der nach einem der Ansprüche 1 bis 3 erhaltenen Benzoesäurederivate der Formel (I) als Konservierungsmittel in der Pharmazie, Kosmetologie und in der Agrar-Nahrungsmittelindustrie.

6. Verwendung der nach einem der Ansprüche 1 bis 3 erhaltenen Benzoesäurederivate der Formel (I) zur Herstellung von Desinfektionszusammensetzungen.

**Claims for the contracting states: BE, CH, DE, FR, GB, IT, LI, LU, NL, SE**

1. Benzoic acid derivatives with the general formula:

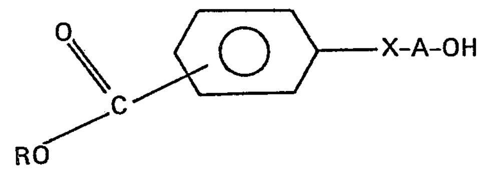

(I)

in which:
- A represents a straight or branched alkyl chain with 5 to 10 carbon atoms,
- X represents the oxygen atom or a direct bond,
- R represents hydrogen or an alkyl group with 2 to 6 carbon atoms, possibly substituted by an alcohol function, and the salts of the said derivatives.

2. Benzoic acid derivatives according to claim 1, characterized in that X is a direct bond, R ist the hydrogen or ethyl group, and A-OH is selected among the groups $(CH_2)_5OH$ and $CH_2CH(OH)n-C_3H_7$.

3. Benzoic acid derivatives according to claim 1, characterized in that X is the oxygen, R is the hydrogen or the ethyl group, and A-OH is the $(CH_2)_5OH$ group.

4. Process for obtaining benzoic acid derivatives according to one of the claims 1 to 3, characterized in that it consists of:

1) subjecting the compound of formula IV to catalytic hydrogenation:

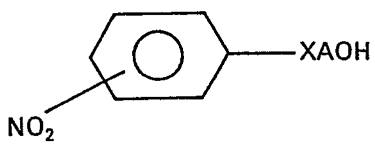

(IV)

in which X and A are as defined in claim 1, to form the corresponding aniline derivatives,

2) the addition, to the said aniline derivatives, of sodium nitrite in acidic medium, to form the corresponding diazoniums,

3) the conversion of the said diazoniums to benzonitriles by the action of cuprous cyanide,

4) the conversion of the benzonitriles obtained to formula I compounds and

5) if necessary, the conversion of the said compound obtained to one of its salts.

5. Pharmaceutical composition for external use displaying antimicrobial activity characterized in that it contains an effective quantity of a compound according to one of claims 1 to 3.

6. Application of derivatives according to one of the claims 1 to 3 as preservative in drugs, cosmetology and agri-foodstuffs.

7. Cosmetic products, characterized in that they contain, as preservatives, a derivative according to any one of claims 1 to 3.

8. Disinfectant compositions, characterized in that they contain an effective quantity of a derivative according to one of the claims 1 to 3.

**Claims for the contracting State: AT**

1. Process for the preparation of benzoic acid derivatives with the general formula:

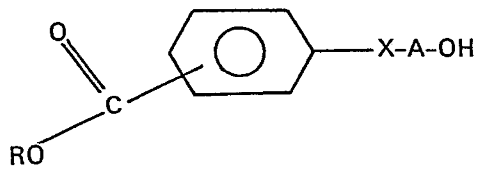

(I)

in which:
- A represents a straight or branched alkyl chain with 5 to 10 carbon atoms,
- X represents the oxygen atom or a direct bond,
- R represents hydrogen or an alkyl group with 2 to 6 carbon atoms, possibly substituted by an alcohol function, and the salts of the said derivatives, characterized in that it consists of:

1. subjecting the compound of formula IV to catalytic hydrogenation:

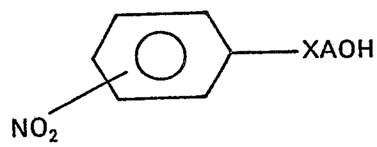

(IV)

in which X and A are as defined in claim 1, to form the corresponding aniline derivatives,

2) the addition, to the said aniline derivatives, of sodium nitrite in acidic medium, to form the corresponding diazoniums,

3) the conversion of the said diazoniums to benzonitriles by the action of cuprous cyanide,

4) the conversion of the benzonitriles obtained to formula I compounds and

5) if necessary, the conversion of the said compound obtained to one of its salts.

2. Process according to claim 1, characterized in that X is a direct bond, R is the hydrogen or ethyl group, and A-OH is selected among the groups $(CH_2)_5OH$ and $CH_2CH(OH)n-C_3H_7$.

3. Process according to claim 1, characterized in that X is the oxygen, R is the hydrogen or the ethyl group, and A-OH is the $(CH_2)_5OH$ group.

4. Use of benzoic acid derivatives of formula (I) obtained according to any one of claims 1 to 3, for the preparation of pharmaceutical compositions for external use displaying antimicrobial activity.

5. Application of benzoic acid derivatives according to one of the claims 1 to 3 as preservative in drugs, cosmetology and agri-foodstuffs.

6. Use of benzoic acid derivatives of formula (I), obtained according to any one of claims 1 to 3, for the preparation of disinfectant compositions.